# EUROPÄISCHE PATENTANMELDUNG

(11) **EP 2 394 605 A1**
(43) Veröffentlichungstag der Anmeldung: **14.12.2011**
(21) Anmeldenummer: 11166866.1
(22) Anmeldetag: 20.05.2011
(51) Int. Cl.: A61F 2/24

(54) **Medizinisches Klappenimplantat zur Implantation in einen tierischen und/oder menschlichen Körper**

(30) Priorität: 09.06.2010 US 352835 P
(71) Anmelder: Biotronik AG, 8180 Bülach (CH)
(72) Erfinder: Le Cerf, Nils, 8702, Zollikon (CH)
(74) Vertreter: Lindner-Vogt, Karin L.

(57) **Zusammenfassung**

Die Erfindung betrifft ein medizinisches Klappenimplantat (10) zur Implantation im tierischen und/oder menschlichen Körper, mit einem Grundkörper (16), der ein erstes Ende (12) und ein zweites Ende (14) aufweist, die in einer Haupterstreckungsrichtung (44) des Grundkörpers (16) an sich gegenüberliegenden Enden des Grundkörpers (16) angeordnet sind.

Es wird vorgeschlagen, dass eine oder mehrere Hilfseinrichtungen (20), die zur Implantation benötigt werden und/oder einen zeitlich begrenzten Funktionszweck haben und die nach der Implantation zum Verbleib im Körper vorgesehen sind, wenigstens bereichsweise aus einem im Körper abbaubaren Material (22) gebildet sind.

## Beschreibung

Die Erfindung betrifft ein medizinisches Klappenimplantat zur Implantation in einen tierischen und/oder menschlichen Körper nach dem Oberbegriff des Patentanspruchs 1.

In der Medizin kommen Implantate zum Einsatz, die zur Erfüllung von Ersatzfunktionen permanent oder zumindest für einen längeren Zeitraum in einen tierischen und/oder menschlichen Körper eingebracht werden. Bekannt sind z.B. Klappenimplantate, wie etwa Aortenklappenimplantate, welche die Funktion der natürlichen Aortenklappe übernehmen.

Bekannt sind beispielsweise Aortenklappen, bei denen das erkrankte Segel zwischen dem Klappenimplantat und Aortenwand eingeklemmt wird, womit eine Einengung des Lumens einhergeht. Ein solches Klappenimplantat in Form einer künstlichen Aortenklappe ist beispielsweise aus der US 7,399,315 bekannt. Das Klappenimplantat weist ein elastisches Grundgerüst auf, das die Klappen trägt und stützt und an dem metallische Positionierkörper angeordnet sind, mit denen das Klappenimplantat an seinem Zielort in der Lumenwand passend zu den natürlichen Klappen verankert wird. Das Grundgerüst ist mit Material überzogen, welches das Anwachsen des Klappenimplantats unterstützen soll.

Ein häufiges Problem bei Klappenimplantaten, insbesondere am Herzen, ist die Kalzifizierung, d.h. die Ablagerung von Kalziumsalzen, insbesondere Kalziumphosphat (Hydroxyapatit). Kalzifizierung kann in und auf einigen Materialien eines medizinischen Implantats auftreten, während sie mit Körperflüssigkeiten des Patienten in Kontakt kommen. Kalzifizierung kann die Leistung und strukturelle Integrität medizinischer Implantate, die aus kalzifizierungsempfindlichen Materialien bestehen, besonders über längere Zeiträume beeinträchtigen. Kalzifizierung ist beispielsweise die Hauptursache für klinisches Versagen bioprothetischer Herzklappen aus porcinen Aortenklappen oder Rinderherzbeuteln. Kalzifizierung tritt besonders stark an Belastungsstellen auf, an denen z.B. eine Naht durch Gewebe läuft. Kalzifizierung beeinflusst zudem erheblich die Leistung von Klappenimplantaten, die aus synthetischen Materialien wie Polyurethan bestehen.

Im Allgemeinen beginnen bioprothetische Herzklappen etwa sieben Jahre nach der Implantation auszufallen, und nur wenige bioprothetische Herzklappen sind nach 20 Jahren noch funktionstüchtig. Durch die Notwendigkeit des Ersetzens eines degenerierenden Klappenimplantats wird der Patient einem zusätzlichen Operationsrisiko ausgesetzt, wobei das Alt-Implantat entfernt werden oder am Implantationsort verbleiben muss.

Der Erfindung liegt die Aufgabe zugrunde, ein medizinisches Klappenimplantat mit verbesserten Langzeit-Eigenschaften zu schaffen.

Die Aufgabe wird erfindungsgemäß durch die Merkmale des Patentanspruchs 1 gelöst. Günstige Ausgestaltungen und Vorteile der Erfindung ergeben sich aus den weiteren Ansprüchen, der Zeichnung und der Beschreibung.

Die Erfindung geht aus von einem medizinischen Klappenimplantat, mit einem Grundkörper, der ein erstes Ende und ein zweites Ende aufweist, die in einer Haupterstreckungsrichtung des Grundkörpers an sich gegenüberliegenden Enden des Grundkörpers angeordnet sind.

Es wird vorgeschlagen, dass eine oder mehrere Hilfseinrichtungen, die zur Implantation benötigt werden und/oder einen zeitlich begrenzten Funktionszweck haben und die nach der Implantation zum Verbleib im Körper vorgesehen sind, wenigstens bereichsweise aus einem im Körper abbaubaren Material gebildet sind. Insbesondere können die eine oder mehrere Hilfseinrichtungen auch vollständig aus einem im Körper abbaubaren Material gebildet sein.

Es wird alternativ oder zusätzlich vorgeschlagen, dass der Grundkörper wenigstens bereichsweise aus einem im Körper abbaubaren Material gebildet ist.

Gemäß einer vorteilhaften Ausgestaltung kann die Hilfseinrichtung eine oder mehrere Positioniereinrichtungen umfassen, die nach der Implantation zum Verbleib im Körper vorgesehen ist, und die wenigstens bereichsweise oder auch vollständig aus einem im Körper abbaubaren Material gebildet sind. Diese Positionierungseinrichtungen können beispielsweise bügelförmig ausgestaltet sein, an einem Ende des Grundkörpers fixiert sein und in ihrer Länge so bemessen sein, dass sie bei Kontaktierung des Annulus die korrekte Positionierung des Grundkörpers gewährleisten.

Denkbar sind auch Hilfseinrichtungen, die der temporären Verankerung des Grundkörpers dienen und diesen fixieren, bis dieser eingewachsen ist. Diese Verankerungseinrichtungen können kranzförmig ausgestaltet sein mit einem Kranzdurchmesser, der den Durchmesser des Grundkörpers an dieser Position überschreitet.

Unter einem "Ende des Grundkörpers" soll hier insbesondere ein Bereich des Klappenimplantats verstanden werden, an den sich nur in einer Richtung eine Implantatstruktur bzw. Grundkörperstruktur, wie beispielsweise ein Drahtgeflecht, anschließt. Hier soll unter einer "Haupterstreckung" insbesondere eine Länge des Grundkörpers und/oder in einem implantierten Zustand des Klappenimplantats eine Richtung entlang einer Fließrichtung eines Fließmediums verstanden werden. Unter "sich gegenüberliegenden Enden" sollen hier insbesondere Enden verstanden werden, die relativ zur Fließrichtung an unterschiedlichen Enden bzw. am Anfang und am Ende des Klappenimplantats angeordnet sind. Durch die erfindungsgemäße Ausgestaltung wird ein medizinisches Klappenimplantat bereitgestellt, das abgestimmt ist auf die Parameter der Implantationsstelle, wie eine Wirkstoffabsorption einer an dem Implantat angrenzenden und/oder anliegenden Hohlraumwand und/oder einer Fließgeschwindigkeit eines durch den Hohlraum bzw. das Implantat durchfließenden Fließmediums.

Ferner soll unter einem "Klappenimplantat" insbesondere ein Körper verstanden werden, der zumindest eine Ersatzfunktion eines Rückschlagventils, permanent oder für einen längeren Zeitraum bei Implantation in einen tierischen und/oder menschlichen Körper erfüllt. Denkbar wären hier sämtliche, dem Fachmann als zweckdienlich erscheinende medizinisehe Klappenimplantate, wie beispielsweise ein Aortenklappe, einer Pulmonalklappe, einer Mitralklappe oder einer Trikuspidalklappe; besonders vorteilhaft wird eine Ausbildung des medizinischen Implantats als ein Stent, insbesondere ein Koronarstent, vorgeschlagen.

Ferner soll in diesem Zusammenhang unter einem "Grundkörper" insbesondere eine Struktur, wie beispielsweise ein Drahtgeflecht, verstanden werden, die im Wesentlichen eine Gestalt und/oder eine Form des Implantats bzw. des Stents bildet. Zudem ist der Grundkörper vorzugsweise aus einem elastischen oder superelastischen Material, etwa einem metallischen Material und/oder aus einer Kombination von mehreren metallischen Materialien gefertigt, wie beispielsweise Eisen, Magnesium, Nickel, Wolfram, Titan, Zirkonium, Niob, Tantal, Zink, Silizium, Lithium, Natrium, Kalium, Kalzium, Mangan und/oder jedem anderen, dem Fachmann als sinnvoll erscheinenden Material. Möglich wären auch eine Zink-Kalziumlegierung und/oder ein Formgedächtnis-Material, wie beispielsweise eine Nickel-Titan-Legierung und/oder Kupfer-Zink-Aluminium-Legierung, vorzugsweise Nitinol. Des Weiteren kann es vorteilhaft sein, wenn der Grundkörper zumindest Kobalt und/oder Chrom aufweist, vorzugsweise in der Form von Edelstahl und/oder eines Cr-Ni-Fe-Stahl - hier bevorzugt die Legierung 316L - oder einem Co-Cr-Stahl. Durch diese Ausgestaltung kann ein Implantat bereitgestellt werden, das zu zufrieden stellenden Beschichtungsergebnissen führt sowie eine gute Dilatierbarkeit und eine vorteilhafte Flexibilität gepaart mit einer hohen Stabilität aufweist.

Grundsätzlich wäre es jedoch auch denkbar, dass der Grundkörper des Klappenimplantats mindestens teilweise aus Kunststoff, einer Keramik und/oder aus einem biodegradierbaren Material besteht.

Günstigerweise kann eine Optimierung von perkutanen Klappenimplantaten hinsichtlich Einwachsverhalten und Lumen erreicht werden. Ein Vorteil, wenn nur zur Implantation notwendige Bereiche des Klappenimplantats wenigstens bereichsweise aus im Körper abbaubarem Material bestehen, liegt weiterhin darin, dass eine unnötige Reizung des umgebenden Gewebes durch derartige Bereiche vermieden werden kann oder zumindest nur temporär besteht und mit der Zeit bis zum völligen Verschwinden abnimmt.

Neben einem besseren Einwachsen des Klappenimplantats in die Lumenwand kann auch ein größeres Lumen am Implantationsort erreicht werden, da sich ein Teil des Klappenimplantats mit der Zeit auflöst und das Volumen des Klappenimplantats damit reduziert wird. Günstig ist eine insgesamt möglichst geringe Wandstärke des Klappenimplantats, um das Lumen nicht einzuschränken und einen mechanischen Reiz auf die Lumenwand zu minimieren.

Um eine akkurate Positionierung des Klappenimplantats zu erreichen, wird bei einigen Designs von Klappenimplantaten mit "Fühlern" gearbeitet, die dem Operateur eine Rückmeldung geben, wann das Klappenimplantat auf dem gewünschten Implantationsort, d.h. dem Annulus, aufliegt. Diese Fühler werden nach Expansion des Klappenimplantats nicht mehr benötigt, verbleiben jedoch am Implantationsort.

Positionierungseinrichtungen stellen redundantes Material dar, das im Körper verbleibt und potentiell zu Irritationen durch mechanische Reize führt, was mit der Zeit eine Kalzifizierung im Bereich des Klappenimplantats fördert und häufig eine Implantation eines neuen Klappenimplantats notwendig macht.

Teile des Klappenimplantats, die beim Zeitpunkt der Implantation hilfreich sind und/oder einen zeitlich begrenzten Funktionszweck haben , degradieren im Laufe der Zeit, verringern das Volumen des Klappenimplantats und tragen nicht mehr zu einer etwaigen Irritation des Implantationsgebiets bei.

Vorteilhaft kann das Material vom Körper resorbierbar sein.

Günstig sind z.B. langsam abbaubare Polymere, wie etwa Polydioxanon, Polyglycolid, Polylactide [Poly-L-Lactid, Poly D,L Lactid, und Copolymere sowie Blends wie Poly(L-Lactid-co-glycolid), Poly(D,L-lactid-co-glycolid), Poly(L-Lactid-co-D,L-Lactid), Poly(L-Lactid-co-trimethylen carbonat)], Poly-ε-caprolacton, Di- und Triblockcopolymere der oben genannten Lactide mit Polyethylenglycol, Polyhydroxyvalerat, Ethylvinylacetat, Polyethylenoxid, Polyphosphorylcholin, Polyhydroxybuttersäure (ataktisch, isotaktisch, syndiotaktisch sowie deren Blends), Polyorthoesther, Polyanhydride, etc.

Ebenso können schnell abbaubare Materialien eingesetzt werden, wie z.B. Saccharide (Alginat, Chitosan, Levan, Hyaluronsäure und Uronide, Heparin, Dextran, Nitrocellulose, Celloloseacetat bzw. Derivate der Cellulose, Maltodextrin, Chondroidinsulfat, Carragenan, etc.), Fibrin, Albumin, Polypeptide und deren Derivate etc.

Das abbaubare Material kann auch Magnesium umfassen. Günstigerweise können ganze Komponenten aus dem abbaubaren Material, z.B. Magnesium, gebildet sein, so dass diese sich vollständig auflösen. So können beispielsweise Positionierhilfen, welche beim Einführen des Klappenimplantats dem Operateur anzeigen, dass der gewünschte Platz erreicht ist, vollständig aus dem abbaubaren Material gebildet sein.

Alternativ oder zusätzlich kann eine ablagerungshemmende, insbesondere kalzifizierungshemmende, Beschichtung vorgesehen sein, insbesondere Homocysteinsäure. Damit kann die Gefahr einer Störung oder eines Funktionsausfalls des Klappenimplantats verringert werden.

Das Klappenimplantat kann vorteilhaft als Aortenklappe ausgebildet sein. Ebenso ist eine Ausgestaltung als Pulmonalklappe oder auch eine Ausgestaltung als Mitralklappe denkbar.

Es können verbesserte klinische Ergebnisse auf Grund eines höheren Lumens und geringerer Gefäßirritationen erreicht werden. Vorteilhaft kann ein Klappenimplantat geschaffen werden, insbesondere eine Aortenklappe, welches in einem ersten Schritt einwachsen kann (temporärer Prozess) und danach nicht mehr durch Metall fixiert wird, sondern durch das eigene Körpergewebe. Dies kann vorteilhaft sein für eine Zweitimplantation eines Klappenimplantats am gleichen Implantationsort, bei dem das neue Klappenimplantat im alten Klappenimplantat platziert wird. Darüber hinaus kann im Fall einer derartigen Aortenklappe erwartet werden, dass Komplikationen wie Störungen der Mitralklappe oder Notwendigkeit eines Herzschrittmachers reduziert werden.

Vorteilhaft kann am Klappemimplantat eine ablagerungshemmende, insbesondere kalzifizierungshemmende, Beschichtung vorgesehen ist, insbesondere Homocysteinsäure. Dabei kann insbesondere die eine oder mehrere Positioniereinrichtungen mit der ablagerungshemmenden, insbesondere kalzifizierungshemmenden, Beschichtung versehen sein.

Die Erfindung ist nachfolgend beispielhaft, anhand von in Zeichnungen dargestellten Ausführungsbeispielen, näher erläutert. Es zeigen in schematischer Darstellung:
- Fig. 1: eine erfindungsgemäßes medizinisches Klappenimplantat im eingebauten Zustand, und
- Fig. 2: eine schematische Darstellung des Einführens einer Aortenklappe an einen Implantationsort.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

Zur Vermeidung unnötiger Wiederholungen wird bei nicht näher beschriebenen Elementen in einer Figur auf die jeweilige Beschreibung der Elemente in vorstehenden Figuren verweisen.

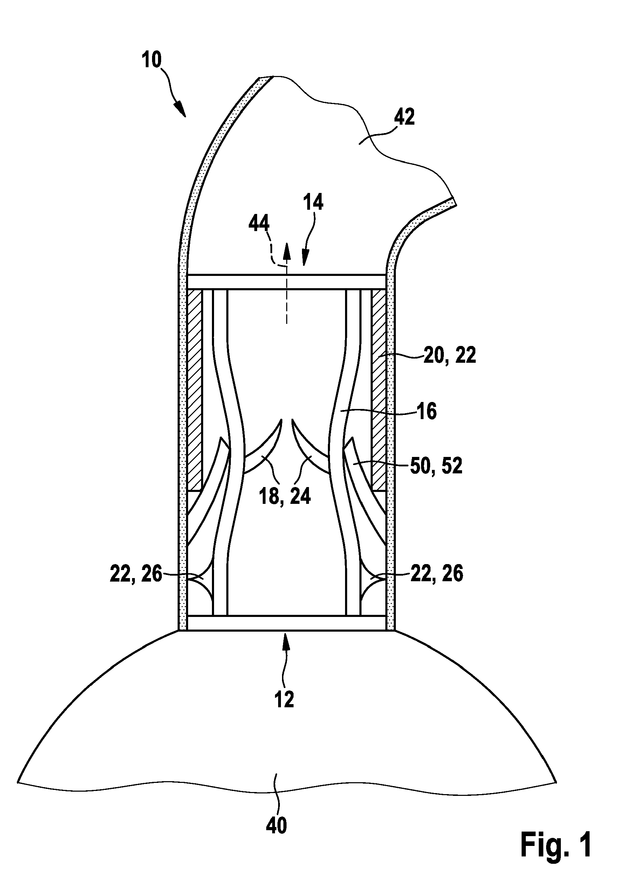

Fig. 1 zeigt in einer Ansicht schematisch ein medizinisches Klappenimplantat 10 in eingebauter Form z.B. im Annulus einer Aortenklappe 50 auf, der in einem Gefäß 42, hier die Aorta, vor dem linken Ventrikel 40 des Herzens angeordnet ist.

Das Klappenimplantat 10 weist z.B. die Form eines Stents auf mit einem aus z.B. Chrom-Kobalt-Stahl gefertigten Grundkörper 16, der als Grundkörperstruktur beispielsweise ein Drahtgeflecht, ausgebildet von Stentstruts, aufweisen kann. Ferner weist der Grundkörper 16 ein erstes Ende 12 und ein zweites Ende 14 auf, die in einer Haupterstreckungsrichtung 44 des Grundkörpers 16 an sich gegenüberliegenden Enden des Grundkörpers 16 angeordnet sind. Generell kann der Grundkörper 16 jede beliebige, dem Fachmann als sinnvoll erscheinende Form aufweisen.

Der Grundkörper 16 ist an seinem stromabwärtigen Ende von einer Positioniereinrichtung 20 umgeben, welches ein im Körper abbaubares Material 22 aufweist, vorzugsweise von diesem gebildet ist. Der Grundkörper 16 drückt die Segel 52 der natürlichen Aortenklappe 50 gegen die Lumenwand des Gefäßes 42 und schafft so einen Freiraum für Segel 18 im inneren Bereich des Grundkörpers 16. Die Segel 18 arbeiten als Rückschlagventil und erlauben einen Blutfluss vom Ventrikel 40 zum Gefäß 42, blockieren jedoch einen Blutfluss in Gegenrichtung. Die Positionierungseinrichtung 20 kann beispielsweise bügelförmig ausgestaltet und an einem Ende des Grundkörpers 16 fixiert sein und in ihrer Länge so bemessen sein, dass sie bei Kontaktierung des Annulus die korrekte Positionierung des Grundkörpers 16 gewährleistet. Die Positioniereinrichtung 20 ist aus im Körper abbaubarem Material 22 gebildet und löst sich mit der Zeit auf.

Die Segel 18 des Klappenimplantats 10 können mit einer Beschichtung 24 versehen sein, welche eine Ablagerung von Kalziumsalzen unterbindet. Während sich die Positioniereinrichtung 20 mit der Zeit auflöst und sich das Lumen des Klappenimplantats 10 dadurch vergrößert, verhindert die Beschichtung 24 eine Kalzifizierung der neuen Segel 18 des Klappenimplantats 10.

Eine weitere Hilfseinrichtung 26 kann der temporären Verankerung des Grundkörpers 16 dienen und diesen fixieren, bis der Grundkörper 16 eingewachsen ist. Diese Verankerungseinrichtungen 26 können kranzförmig ausgestaltet sein mit einem Kranzdurchmesser, der den Durchmesser des Grundkörpers 16 an dieser Position überschreitet. Die Verankerungseinrichtungen 26 sind aus im Körper abbaubarem Material 22 gebildet und lösen sich mit der Zeit auf.

In einem zeichnerisch nicht ausgeführten Ausführungsbeispiel können zusätzlich oder alternativ auch Bereiche des Grundkörpers 16 selbst aus einem im Körper abbaubaren Material 22 gebildet sein.

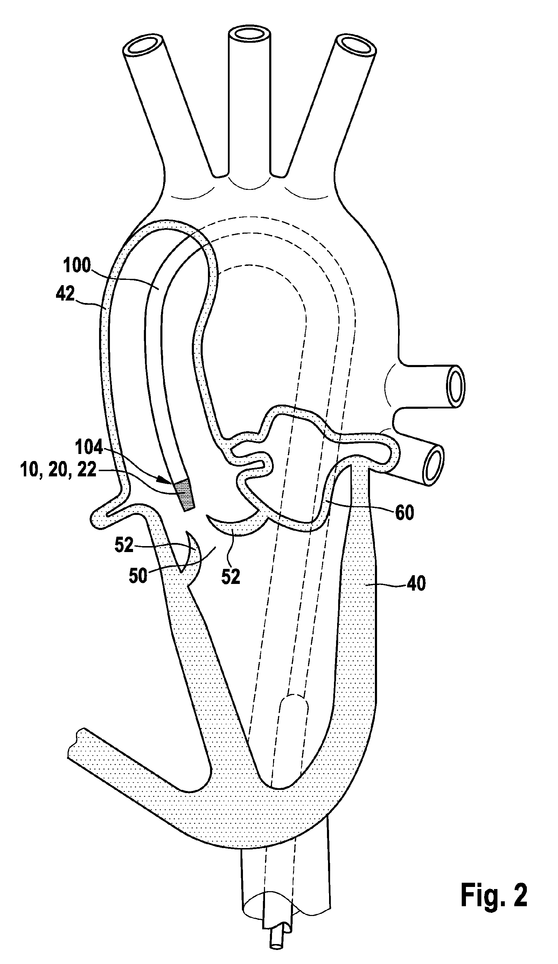

In der Fig. 2 ist das Einführen des medizinischen Klappenimplantats 10 schematisch in einer Teilschnittdarstellung illustriert. Das Klappenimplantat 10 wird in komprimiertem Zustand an einer Spitze 104 eines Katheters 100 in an sich bekannter Weise an den Implantationsort, z.B. den Annulus der Aortenklappe 50 zugeführt. Um die aktuelle Position des Klappenimplantats 10 verfolgen zu können und das Klappenimplantat 10 am gewünschten Implantationsort positionieren zu können, ist am Klappenimplantat 10 eine, z.B. metallische, Positioniereinrichtung 20 angeordnet, die mit dem Klappenimplantat 10 aus dem Katheter 100 ausgestoßen wird.

Das Klappenimplantat 10 ist in diesem Ausführungsbeispiel eine Aortenklappe, welche nach dem Einsetzen am Implantationsort in einem ersten Schritt einwachsen kann (temporärer Prozess). Während sich die z.B. metallische Positioniereinrichtung 20 und/oder Verankerungseinrichtung 26 mit der Zeit auflöst, ist das Klappenimplantat 10 danach nicht mehr durch Metall fixiert, sondern durch das eigene Körpergewebe. Dies ist vorteilhaft für eine Zweitimplantation eines Klappenimplantats am gleichen Implantationsort, bei dem das neue Klappenimplantat im alten Klappenimplantat 10 platziert wird. Komplikationen wie z.B. Störungen der Mitralklappe 60 oder eine Notwendigkeit eines Herzschrittmachers können günstigerweise reduziert werden.

## Patentansprüche

1. Medizinisches Klappenimplantat (10) zur Implantation im tierischen und/oder menschlichen Körper, mit einem Grundkörper (16), der ein erstes Ende (12) und ein zweites Ende (14) aufweist, die in einer Haupterstreckungsrichtung (44) des Grundkörpers (16) an sich gegenüberliegenden Enden des Grundkörpers (16) angeordnet sind, **dadurch gekennzeichnet, dass** eine oder mehrere Hilfseinrichtungen (20), die zur Implantation benötigt werden und/oder einen zeitlich begrenzten Funktionszweck haben und die nach der Implantation zum Verbleib im Körper vorgesehen sind, wenigstens bereichsweise aus einem im Körper abbaubaren Material (22) gebildet sind.

2. Medizinisches Klappenimplantat (10) zur Implantation im tierischen und/oder menschlichen Körper, mit einem Grundkörper (16), der ein erstes Ende (12) und ein zweites Ende (14) aufweist, die in einer Haupterstreckungsrichtung (44) des Grundkörpers (16) an sich gegenüberliegenden Enden des Grundkörpers (16) angeordnet sind, **dadurch gekennzeichnet, dass** der Grundkörper (16) wenigstens bereichsweise aus einem im Körper abbaubaren Material (22) gebildet ist.

3. Medizinisches Klappenimplantat nach Anspruch 1 oder 2, **dadurch gekennzeichnet, dass** eine oder mehrere Hilfseinrichtungen (20) wenigstens eine Positioniereinrichtungen (20) umfassen, die nach der Implantation zum Verbleib im Körper vorgesehen sind, und welche wenigstens bereichsweise aus einem im Körper abbaubaren Material (22) gebildet sind.

4. Medizinisches Klappenimplantat nach Anspruch 1 oder 2, **dadurch gekennzeichnet, dass** die eine oder mehrere Hilfseinrichtungen (22) wenigstens eine Verankerungseinrichtung (26) umfassen, die nach der Implantation zum Verbleib im Körper vorgesehen sind, und welche wenigstens bereichsweise aus einem im Körper abbaubaren Material (22) gebildet sind.

5. Medizinisches Klappenimplantat nach einem der vorhergehenden Ansprüche, **dadurch gekennzeichnet, dass** das abbaubare Material (22) ein Polymer umfasst, insbesondere eines aus der Gruppe Polydioxanon, Polyglycolid, Polylactide [Poly-L-Lactid, Poly D,L Lactid, und Copolymere, sowie Blends wie Poly(L-Lactid-co-glycolid), Poly(D,L-lactid-co-glycolid), Poly(L-Lactid-co-D,L-Lactid), Poly(L-Lactid-co-trimethylen carbonat)], Poly-ε-caprolacton, Di- und Triblockcopolymer der oben genannten Lactide mit Polyethylenglycol, Polyhydroxyvalerat, Ethylvinylacetat, Polyethylenoxid, Polyphosphorylcholin, Polyhydroxybuttersäure (ataktisch, isotaktisch, syndiotaktisch sowie deren Blends), Polyorthoesther, Polyanhydride.

6. Medizinisches Klappenimplantat nach einem der vorhergehenden Ansprüche, **dadurch gekennzeichnet, dass** das abbaubare Material (22) Magnesium umfasst.

7. Medizinisches Klappenimplantat nach einem der vorhergehenden Ansprüche, **dadurch gekennzeichnet, dass** eine ablagerungshemmende, insbesondere kalzifizierungshemmende, Beschichtung (24) vorgesehen ist, insbesondere Homocysteinsäure.

8. Medizinisches Klappenimplantat nach Anspruch 7, **dadurch gekennzeichnet, dass** die eine oder mehrere Positioniereinrichtungen (20), mit der ablagerungshemmenden, insbesondere kalzifizierungshemmenden, Beschichtung (24) versehen ist.

9. Medizinisches Klappenimplantat nach einem der vorhergehenden Ansprüche, **dadurch gekennzeichnet, dass** der Grundkörper (16) einen selbstexpandierenden Stent umfasst.

10. Medizinisches Klappenimplantat nach einem der vorhergehenden Ansprüche, **dadurch gekennzeichnet, dass** der Grundkörper (16) ein elastisches und/oder superelastisches Material aufweist.

11. Medizinisches Klappenimplantat nach einem der vorhergehenden Ansprüche, **gekennzeichnet durch** eine Ausgestaltung als Aortenklappe (50).

12. Medizinisches Klappenimplantat nach einem der vorhergehenden Ansprüche, **gekennzeichnet durch** eine Ausgestaltung als Pulmonalklappe oder Mitralklappe oder Trikuspidalklappe.
